# EUROPEAN PATENT APPLICATION

(11) **EP 2 058 016 A1**
(43) Date of publication of application: **13.05.2009**
(21) Application number: 05717216.5
(22) Date of filing: 16.03.2005
(51) Int. Cl.: A61M 1/02

(54) **DEVICE AND SYSTEM FOR MONITORING SERUM BAG LEVELS**

(71) Applicant: Tamargo S.C., 33203 Gij n, Asturias (ES)
(72) Inventor: PUERTA TAMARGO, Alejandro, E-33203 Gij n, Asturias (ES); FERNANDEZ FERNANDEZ, Maria Belen, E-33203 Gij n, Asturias (ES)
(74) Representative: Falcon Morales, Alejandro
(86) International application number: PCT/ES2005/000138
(87) International publication number: WO 2006/097546

(57) **Abstract**

Device and system for monitoring the level of serum bags. This system includes an electronic device which is protected by a casing having upper a lower faces with respective hooks (3, 5). A bag or bottle (2) is suspended from one hook (3). The electronic device comprises: a charge cell (11) which supplies an electric signal that is proportional to the weight supported by the hook (3), an amplifier (12) and an analogical/digital converter (13) which forwards the acquired signal to a microcontroller (14). This microcontroller calculates the quantity of liquid as a function of the electric signal, manages the pressing to the push a bottom (7), transmits information to the screen (4) and activates alarms when a programmed value is reached. In addition to this, the microcontroller (14) is equipped with an integrated communication port that facilitates communication between the device and the central monitoring system or other devices.

## Description

### Field of the invention.

This invention can be placed within area for monitoring the emptying and filling of liquids in deposits, more concretely for monitoring the emptying and filling liquids in serum bags and bottles for the sanitary sector.

### Background of the invention.

In hospital, clinics and other health institutions, sanitary workers monitor themselves filling and emptying of serum or solutions in bottles and bag. This task takes time and resources and the more bags they have to supervise, the more risk the worker assume and this situation could became fatal for some patients.

On the other hand, organic serum supply is specially important in the postoperative for those people who underwent certain surgery. In theses cases, monitoring becomes a critical task which can demand most of the resources in a unit during the postoperative period.

### Summary of the invention.

This invention aims to monitor the level of serum or other medical substance in a bag or bottle when this substance is extracted or introduced in a patient in a continuous way. This monitoring system consist of placing an electronic measure device between the holder of the serum bag or bottle and the bag or bottle itself. Then, the electronic measuring device hangs on the holder which was initially foreseen to hang the serum bottle or bag and in turn, the bag or bottle hangs on the electronic measuring device. The electronic device measures the weight of the bag or bottle and the voltage of the battery which provides energy to this device. Later on, the electronic measuring device sends a signal to a central monitoring system which centralizes the recorded measures by a network of the electronic devices. This way, we keep the measures recorded by each of the devices in one only point. Each electronic device is powered by a battery or by a conventional tension adapter connected to power supply.

There are some different kinds of systems to communicate the electronic measuring devices to a central monitoring device. We have chosen a radiofrequency system based on the frequency of 433,92 MHz that is the one foreseen in European Union (EU) for this type of applications. In the event of being used in a country not belonging to the EU, it could be necessary to change the frequency according to their legislation. According to the special conditions of the sanitary center it could be possible to use another communication system different from the one proposed, e.g. RS-485/422, infrareds, Ethernet, and other well-known methods in the state of the art. Those systems could even be more efficient than the one proposed of radio frequency. Nevertheless, this system presents the advantage of being able to be applied in most cases since it needs neither auxiliary elements nor specific conditions.

The proposed electronic measuring device is a square prism in shape. Nevertheless, this devices could be made in other shapes.

Its superior face has:
- a hook or superior anchorage that it is hooked to the support which initially was foreseen to hang the bag or bottle
- an antenna which transmits and to receives data from the central system of supervision. The antenna is external due to the better efficiency although an internal antenna is possible. The working frequency is 433,92.

Its lower face has another hook or anchorage that it is good to support the bottle or bag. When the bag or bottle is hanged on the hook of the electronic device, it measures the weight of the bag using a load cell which provides a proportional electrical signal to the weight. An instrumentation amplifier duly raises this signal to a required level for the digital analogical converter. A microcontroller manages the signals which arrive at the analogical digital converter. These signals correspond to the taken measures which are measured every two seconds These measured data is recorded in a memory which keeps the last ten recorded measures. The microcontroller calculates the average measure from theses ten measures but it does not take into account the highest and the lowest values. Then, the resulting value is proportionately to remaining liquid in the bag.

The measuring device works with any kind of bag or bottle but the hooks and bags impose the maximum weight for working. We must calibrate the device when a new kind of bag is used for the first time. Therefore, first the full bag or bottle is hang from the hook of the device and second we hang the bag or bottle from the hook once the bag or bottle is empty. The device records these weights and it calculates the remaining liquid in the bag or bottle taking into account the differences between the measured weights. The measures are carried out to regular intervals of time and the device calculates the remaining liquid according to the measured weight. In the event of the measured weight is higher or equal to the established one as full bag, the device ascribes 255 to this measure. If the measured weight is less or equal to the established one as empty bag, the device will ascribe 0 to this measure. For every measure weight between full and empty bag is assigned a entire proportional value in between 0 and 255 according to the remaining liquid in the bag.. By this way, we translate the measure in binary base using less than 8 digits recording this value in one byte in the microcontroller memory.

The device will only be calibrated if bags or bottles with different capacities are used or if the weight of the empty bag is different from those used before.

The information arrives the microcontroller of this electronic device from a treated signal that previously has been detected by a load cell and it proceeds:
- To filter and to average out the measures of the sensor that allows to eliminate possible alterations produced by the patient's movements, oscillations of the bag, etc.
- To manage the alarms, including the emission of sound or luminous warnings when the level of liquid higher or lower to a specific value.
- To communicate the data recorded by every measuring device in order to centralize all the measures. This communication is made by a communications protocol of the central system of supervision and using a cable or wirelessly.
- To calculate the remaining time to change the bags or bottles taking into account the variation of weight.

With the previous characteristics, the control of the level in bags and bottles is made easier for the health workers in such a way that these workers will take charge of changing the bag or bottle when the central system warns it or the alarms of each one of the monitoring devices are activated.

The user can trigger or deactivate the alarms, as well as to establish the measure values starting from which the alarm are activated.

It is possible to use the measure devices independently since they are able to manage alarms. Nevertheless, if the central monitoring system is not used, we will not have a point where all measures were collected and shown.

The central monitoring system consist basically of a computer which has a signal transmitter - receiver. The central computer asks each device to get information about the situation of every bag using the transmitter receiver device Every electronic device has an unique identifier because all device work on the same frequency. Then, when the central computer asks for information, it sends a 433,92 MHz signal which arrives to every one of the devices but only one device answer, this one which contains the identifier corresponding to this one contained in the signal emitted by the management central system.

This same way, it is possible to set the triggering alarm values in the control system as well as if the alarms are activated or disabled in each one of the devices in an independent way.

The operation would be similar in the case of using a communication method not based on the radio frequency.

### Brief description of the drawings.

### The following figures are shown to make the invention be understood easer:

Figure 1 shows the electronic monitoring device and the bag or bottle.

Figure 2 is an outside view of the measuring electronic device.

Figure 3 shows a group of the monitoring level system for bottles and bags.

Figure 4 is a basic outline of the operation system.

Detailed description of an illustrative embodiment.

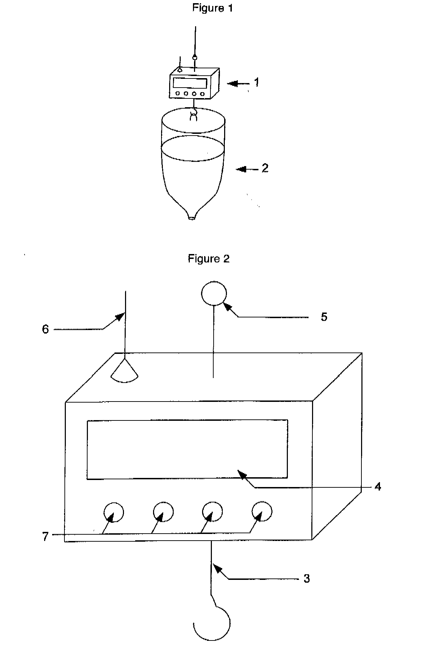

Figure 1 shows the electronic measuring device (1) hung on its hook (5) on a holder not shown. In turn, a serum bag (2) hangs on the measuring electronic device (1) and the device (1) is in between the first holder, not shown, an the bag or bottle (2).

As shown in figure 2, the device (1) has a quadrangular prysma shape. The antenna (6) and the hook (5) are located on the top side of the electronic device (1) and this electronic measuring device (1) hangs on the hook (5). We have chosen an external antenna because it has a bigger efficiency than an internal antenna. The working frequency is 433,92 MHz. Another hook (3) is on the inferior side and the used of this hook (3) is to hang the serum bag or bottle (2). The electronic device (1) measures the weight of the bag or bottle (2) when the bag or bottle (2) is hang on the hook (3). Once the weight is measured, the electronic device (1) shows the level of liquid according to the weight. A display (4) which shows the remaining volume in the bag of bottle (2) is located on one face of the device. But before measuring, the device should be calibrated. Then, the user will calibrate the electronic device (1) with the help of the information shown on the display (4) and some buttons (7). The user will define the alarms for the battery, for the emptying and filling levels. The user calibrates the device weighting first the full or almost full bag (2) a later the empty or almost empty bag or bottle (2). According to the user whishes, the microcontroller assigns 255 to the weight of the bag when is full or almost full and it assigns 0 to the weight of the bag when is empty or almost empty. Then, with this data, the emptying or filling procedure can be graduated. In the event of the measured weight is higher or equal to the established one as a full bag, the device will assign 255 to this measure. If the measured weight is less or equal to the established one as an empty bag, the device will assign 0 to this measure. According to the remaining liquid in the bag, for every measured weight is assigned a entire proportional value in between 0 and 255 as far as this measured weight is between the value of a full and an empty bag. Then the electronic measuring device (1) is composed by a microcontroller which can be calibrated and programmed in order to get the characteristic of different kind of bags or bottles (2) which are hang on the hook (3). Finally, the microcontroller records the information detected by the sensor and:
- To filter and to average out the measures taken by the sensor in order to eliminate possible alterations.
- To manage the alarms, including the emission of sound or luminous warnings when the level of liquid is higher or lower to a specific value.
- To communicate the data recorded by every measuring device in order to centralize all the measures. This communication is made by a protocol of communications of the central system of supervision and using a cable or wirelessly.
- To calculate the remaining time to change the bags or bottles taking into account the variation of weight.

A voltage adapting system or a battery supplies energy to the electronic device (1). Neither voltage adapting system nor a battery is shown in figures.

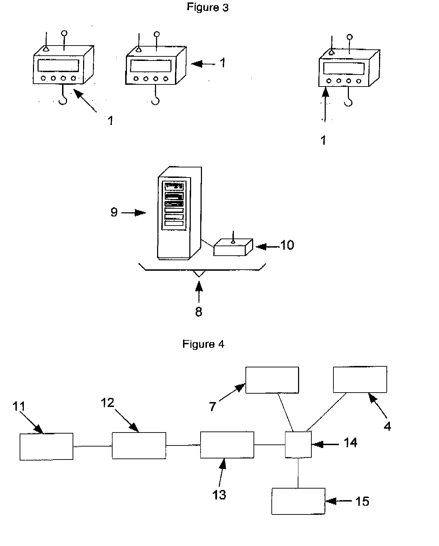

A load cell (11) measures the weight as figure 4 shows. This load cell (11) produces a proportional electrical signal to the weight. An instrumentation amplifier rise voltage to the required level of the analogical digital converter. (13) The instrumentation amplifier (12) gives a 0 volts value when weight I equal to zero and 5 volts for the maximum weight. Nevertheless, this last voltage depends on the voltage of the analogical digital converter. The microcontroller (14) manages this last signal which corresponds to recorded measures. Therefore, every two seconds it records a measure and it keeps it in memory (1) the last ten records. The microcontroller calculates the averages value with these 10 records but not taking into account the highest and lowest value. Then, the calculated value is proportional to the remaining quantity of liquid in the bag or bottle (2).

The analogical digital converter (13) is also used to monitor the voltage of the battery and then to calculate the charge level of the battery. The microcontroller manages the answers from buttons (7) from its digitals entries and exits and the microcontroller sends information to the display (4) and activates the alarm warning. These warning may be buzzer warning or lighting warning.

Once the electronic device (1) measures the weight of the bag (2), it sends a signal to the monitoring central system as figure 3 shows This way measures got by each one of the devices (1) are collected in an only point.

The monitoring level system (8) basically consists of a computer (9) signal transmitter receiver (10). Every electronic device (1) has a unique identifier because all electronic device work at the same frequency and all of them received simultaneously request for sending data. The computer (9) asks devices (9) to obtain information of the liquid level in bags (2) using emitter receiver devices. When computer (9) asks for information, it sends a 433,92 MHz signal by the emitter - receiver (10) and this signal includes the identification code for the device (1). This signal arrives all devices (1) but only the device with the code identifier which is equal to this one included in the signal emitted by the central monitoring system will answer.

The communication between the devices and the central system of supervision (8) is carried out through the communications port. We have chosen a serial port which is integrated in the microcontroller (14) and a standar conector is avalaible for a computer. Signals emitted by every communication port are converted into radiofrequency signals. The emitter-receptor (10), which is connected to the central monitoring system (8), and the device (1) reset the original electrical signals from these radiofrequency signals and they send these reset signals to the serial communication port of the computer (9) or serial port of the microcontroller (14) which manages the device (1).

On the other hand, the user may define the triggering alarm values using the monitoring central system (88) or using the buttons (7). These values should trigger the intensity of signal and the kind of warning, lighting or buzzing. In other words, warnings may be triggered in the central monitoring system (8) or in every device (1). The warning may be lighting or buzzing warning. For this purpose, the microcontrollers (14) of every electronic measuring device (1) activates buzzing warning or lighting warning when the measured weight is higher or lower than the values defined by the user in the monitoring system (8).

Finally, every device (1) may configure its identifier code which characterizes the device within the communication networking and the triggering warning.

Industrial application of the invention.

This invention is specially intended for medical and veterinarian science. For monitoring the emptying and filling of product in human or animal bodies. Generally, industrial application of this invention could be for the monitoring and emptying products in packaging.

## Claims

1. An electronic device that measures the level of liquid in bags and bottles that contain medical substances to be continuously introduced or extracted from a human body wherein this device is made by a superior hook (5), another hook (3) in the inferior face to hang a bag or bottle(2), a load cell (11) to provide an electric proportional sign to the weight that supports the hook (3), an instrumentation amplifier (12) to elevate the tension of the electric signal so that an analogical digital converter (13) remits the acquired signal to the microcontroller (14) that first calculates the quantity of liquid in the bag (2), second manages the pulsations of the buttons (7), third sends information to the screen (4) and fourth activates sound and luminous token in the case of the value programmed through some buttons (7) and a port of communications integrated in the microcontroller (14) so that the device communicates, a central monitoring (8) system or another devices by cable or wireless means.

2. Electronic measure device according to claim 1 wherein the microcontroller manages the measures and it keeps a record of the latest measures and it calculates the weight of the bag and the remaining time to change the bag (2) taking into account the last ten measures and discarding from these last ten measures the highest and the lowest data from the array.

3. Electronic measure device according to claims 1 and 2 wherein the analogical - digital converser (13) monitors the voltage of the battery and it sends a signal to the microcontroller which severs to calculate the battery charge level

4. Electronic measure device according to claims 1 and 2 wherein the communication port which is integrated in the microcontroller is a serried port (14) and it converts an electronic signal in a radifrecuencie signal and emits this signal by an antenna.

5. Electronic measure device according to claims 4 wherein the user calibrate the electronic device (1) making use of the display (4) and buttons (7) ascribing the value 255 to the weight of the full bag and the value 0 to the weigh of the empty or almost empty bag and the user also defines the alarms of the battery level and the level of the bag (2) when is empty or filled.

6. Electronic measure device (1) according to claim 5 **characterized** because once the device (1) is calibrated, the microcontroller (14) ascribes in each moment a numeric value among 0 and 255 proportionally to the measured weight of the bag (2) or bottle.

7. Monitoring level system for bags or bottles that contain medical substances to be introduced or to extracted continuously from humans or animals where the system is composed by a group of devices (1) according to claims 1 at 6 wherein the central monitoring system (8) centralizes the signald received by each one of the devices (1).

8. Monitoring level system for bags or bottles according to claim 7 wherein the monitoring central system (8) consist of a computer (9) with a 433,92 MHz electromagnetic signal transmitter receiver where each of the device (1) has a unique identifier in such a way that the computer (9) asks each device emitting a signal which includes the identifier code and answering only the associated to that identifier code.

9. Monitoring level system for bags or bottles according to claim 8 wherein in each device is configured the alarms and the tokens and the identifier that characterizes it in the communications network.

10. Supervisor level system for bags or bottels according to claim **9** wherein **in the monitoring system (8)** the user **sets** the measure values starting from which the alarms are activated for each device (1) and where **tokens** are activated
